# EUROPEAN PATENT APPLICATION

(11) **EP 2 559 339 A1**
(43) Date of publication of application: **20.02.2013**
(21) Application number: 11768783.0
(22) Date of filing: 07.04.2011
(51) Int. Cl.: A01N 59/00, A01N 25/00, A01P 3/00, A61L 2/20, C02F 1/78

(54) **DISINFECTION AID FOR OZONE DISINFECTION AND METHOD FOR OZONE DISINFECTION**

(30) Priority: 12.04.2010 JP 2010091627
(71) Applicant: Lion Corporation, Tokyo 130-8644 (JP)
(72) Inventor: TAKEUCHI, Yoshikuni, Tokyo 130-8644 (JP); NISHIMURA, Sonoko, Tokyo 130-8644 (JP); WATANABE, Shinnichi, Tokyo 130-8644 (JP); HAYASHI, Aiko, Tokyo 130-8644 (JP); HASEGAWA, Takayuki, Tokyo 130-8644 (JP); KUBOZONO, Takayasu, Tokyo 130-8644 (JP)
(74) Representative: Denjean, Eric
(86) International application number: PCT/JP2011/058821
(87) International publication number: WO 2011/129262

(57) **Abstract**

The present invention provides a sterilization auxiliary for ozone sterilization which is an aqueous solution including a Component (A) of an aluminum compound which produces aluminum ions in an aqueous solution, and a Component (B) of one or more of acids selected from phosphoric acid, citric acid, malic acid, succinic acid, gluconic acid, lactic acid, and L-tartaric acid, wherein the pH of the aqueous solution is greater than or equal to 1.0 and less than 5.0, and an ozone sterilization method for ozone-treating a substance to be treated using the sterilization auxiliary. According to the present invention, a sterilization auxiliary for ozone sterilization in which a high sterilizing effect may be obtained using less ozone without burdening sterilization apparatus and a substance to be treated, and a low-cost ozone sterilization method using the sterilization auxiliary can be provided.

## Description

### Technical Field

The present invention relates to a sterilization auxiliary for ozone sterilization and an ozone sterilization method.
Priority is claimed on Japanese Patent Application No. 2010-091627, filed April 12, 2010, the content of which is incorporated herein by reference.

### Background Art

In recent years, there has been a demand for safer and more reliable sterilization treatments in the sterilization of fresh food and medical equipment, or the stationary sterilization cleaning of an industrial plant line (such as CIP sterilization cleaning). Among these, ozone has been receiving attention as in addition to having a strong oxidizing power and being capable of being made from oxygen, ozone returns to oxygen without remaining as ozone after sterilization. Examples of ozone sterilization methods generally include, a method in which ozone gas is aerated into a substance to be treated which is immersed in water to be treated (an ozone aeration method), and a method in which a substance to be treated is immersed in ozone water in which ozone is dissolved in water (an ozone water immersion method) may be included.

The sterilizing effect of ozone is proportional to its concentration, therefore, the amount of ozone used is inevitably increased in order to obtain a sufficient sterilizing effect. Meanwhile, the ozone concentration in the atmosphere of a working environment is required to be less than or equal to 0.00001 % by volume. As a result, in order to achieve both a sterilizing effect and safety, additional equipment, such as an ozone removal device being installed so that the ozone concentration in the atmosphere of a working environment does not become too high, is often required. In addition, if the amount of ozone used is increased, a larger ozone generator is required, and therefore the cost increases. In addition, the rubber, metal, plastic, and the like, of each unit which is in contact with ozone in the sterilization apparatus have a tendency to corrode, and the load on the apparatus also increases.

Therefore, various attempts have been made to obtain a high sterilizing power using a small amount of ozone by improving the sterilizing effect of ozone.
For example, in an ozone aeration method, (i) a method in which a substance to be treated is washed by ozone gas being aerated into a treatment liquid which includes an agent having a specific dynamic surface tension such as monoacetin, diacetin or triacetin (Patent Document 1), and (ii) a method in which a substance to be treated is washed by ozone gas being aerated to a treatment liquid which further includes water-soluble acid in addition to the above agent (Patent Document 2) have been disclosed. According to methods (i) and (ii), the sterilizing effect is improved due to high contact efficiency with a substance to be treated since the air bubbles formed by the aeration of ozone gas can be miniaturized, therefore, the floating speed of the above air bubbles is reduced, and retention time in the treatment liquid increases.
In addition, in an ozone water immersion method, (iii) a method in which a substance to be treated is washed by a treatment liquid including a glycerin fatty acid ester such as Monocaprylin or Monocaprin, and ozone (Patent Document 3) has been disclosed. According to the method (iii), the sterilizing effect is improved by an organic peroxide which has excellent sterilizing power and can be stably present in a treatment liquid generated from the reaction of a glycerin fatty acid ester and ozone.

### Citation List

### Patent Literature

[Patent Document 1] PCT International Publication No. WO07/040260
[Patent Document 2] Japanese Unexamined Patent Application, First Publication No. 2008-201992
[Patent Document 3] Japanese Unexamined Patent Application, First Publication No. 2008-255045

### Summary of Invention

### Technical Problem

However, in methods (i) to (iii), the sterilizing effects are still not satisfactory, therefore, further improvements in the sterilizing effect have been required.
An object of the present invention is to provide a sterilization auxiliary for ozone sterilization in which a high sterilizing effect may be obtained using less ozone without burdening sterilization apparatus and a substance to be treated, and a low-cost ozone sterilization method using the sterilization auxiliary.

### Solution to Problem

The present invention employs the following configurations in order to solve the above problems.
[1] A sterilization auxiliary for ozone sterilization which is an aqueous solution including a Component (A) of an aluminum compound which produces aluminum ions in an aqueous solution, and a Component (B) of one or more acids selected from phosphoric acid, acetic acid, citric acid, malic acid, succinic acid, gluconic acid, lactic acid, and L-tartaric acid, wherein the pH of the aqueous solution is greater than or equal to 1.0 and less than 5.0.
[2] The sterilization auxiliary for ozone sterilization according to [1], wherein the Component (B) is one or more acids selected from the group consisting of phosphoric acid, citric acid and acetic acid.
[3] The sterilization auxiliary for ozone sterilization according to [1] or [2], wherein the Component (A) is one or more aluminum compounds selected from the group consisting of aluminum potassium sulfate (AlK(SO₄)2·12H₂O), burnt potassium alum (AlK(SO₄)₂), aluminum ammonium sulfate (AlNH₄(SO₄)₂·12H₂O) and burnt ammonium alum (AlNH₄(SO₄)₂).
[4] The sterilization auxiliary for ozone sterilization according to any one of [1] to [3], further including a Component (C) of a glycerin fatty acid ester in which a fatty acid having 1 to 10 carbon atoms and glycerin are ester-bonded.
[5] An ozone sterilization method for ozone-treating a substance to be treated using the sterilization auxiliary for ozone sterilization according to any one of [1] to [4]. Advantageous Effects of Invention

If the sterilization auxiliary for ozone sterilization in an aspect of the present invention is used, a high sterilizing effect may be obtained with less ozone.
Furthermore, according to the ozone sterilization method in another aspect of the present invention, the cost may be reduced since the use of additional facilities is not required, and also the load on the sterilization apparatus and the load on the substance to be treated may be reduced by using the sterilization auxiliary for ozone sterilization of the present invention since a high sterilizing effect may be obtained with less ozone. Brief Description of Drawings

FIG. 1 is a schematic diagram showing one example of sterilization apparatus used in an ozone sterilization method of the present invention.
FIG. 2 is a schematic diagram showing ozone aeration means used in Examples 1 to 12 and Comparative Examples 1 to 11.
FIG. 3 is a schematic diagram showing sterilization apparatus used in Examples 13 to 32 and Comparative Examples 12 to 21.
FIG. 4 is a schematic diagram showing sterilization apparatus used in Examples 33 to 56 and Comparative Examples 22 to 31.

### Description of Embodiments

### [Sterilization Auxiliary]

A sterilization auxiliary for ozone sterilization of the present invention is an aqueous solution including a Component (A) of an aluminum compound which produces aluminum ions in an aqueous solution, and a Component (B) of one or more acids selected from phosphoric acid, acetic acid, citric acid, malic acid, succinic acid, gluconic acid, lactic acid, and L-tartaric acid, wherein the pH of the aqueous solution is greater than or equal to 1.0 and less than 5.0. The sterilization auxiliary of the present invention is preferably used in the sterilization of a substance to be treated by an ozone aeration method using ozone aeration. However, the sterilization auxiliary of the present invention may also be used in the sterilization of a substance to be treated by an ozone water immersion method using ozone water in which ozone is dissolved.

(Component (A): Aluminum Compound which Produces Aluminum Ions in Aqueous Solution)
The Component (A) is an aluminum compound which produces aluminum ions in an aqueous solution.

Examples of the Component (A) may include the following Components (A₁) to (A₄).
Component (A₁): a complex salt including aluminum.
Component (A₂): aluminum salts other than the above complex salt.
Component (A₃): a polymerized compound of aluminum salts.
Component (A₄): an aluminum-containing metal.

Examples of the component (A₁) may include aluminum potassium sulfate (potassium alum, AlK(SO₄)₂-12H₂O), burnt potassium alum (AlK(SO₄)₂), aluminum ammonium sulfate (ammonium alum, AlNH₄(SO₄)₂·12H₂O) and burnt ammonium alum (A1NH₄(SO₄)₂), or the like.
Example of the component (A₂) may include aluminum chloride, chloro hydroxy aluminum, aluminum sulfate (sulfate band), aluminum hydroxide, aluminum phosphate, aluminum silicate, aluminum salts of organic acids (for example, aluminum salts of acetic acid, lactic acid, citric acid, adipic acid, malic acid, succinic acid, maleic acid, fumaric acid, gluconic acid, tartaric acid, glutaric acid, oxalic acid, or the like), aluminum salts of water-soluble chelating agents having an acidic group (an acid dissociable functional group) (for example, aluminum nitroso triacetate, aluminum ethylenediaminetetraacetate, aluminum methyl glycine diacetate, or the like), or the like.
Examples of the Component (A₃) may include polyaluminum chloride, aluminum polyphosphate, or the like.
Example of the Component (A₄) may include aluminum oxide (alumina), pure aluminum, an aluminum alloy (duralumin, or the like), or the like.
The Component (A) may be used either alone or as a combination of two or more.

In the Component (A), it is preferable that the aluminum salts of the Components (A₁) and (A₂) be added since a high sterilizing effect is readily obtained, it is more preferable that the Component (A₁) be added, and particularly, if a substance to be treated is food, it is preferable that one or more aluminum compound selected from the group consisting of aluminum potassium sulfate, burnt potassium alum, aluminum ammonium sulfate and burnt ammonium alum which are food additives be added.

The content of the Component (A) in the sterilization auxiliary of the present invention is preferably 1 to 1,000 mg/L and more preferably 10 to 500 mg/L. If the content of the Component (A) is less than 1 mg/L, it is difficult to obtain a high sterilizing effect. If the content of the Component (A) is greater than 1,000 mg/L, the ozone supplied in the sterilization auxiliary is wasted and it is difficult to obtain an appropriate sterilizing power for the amount of ozone supplied since the aluminum and the ozone present in the sterilization auxiliary react and become aluminum oxide. Furthermore, although it also depends on the amount of ozone supplied, cloudiness and precipitation may form in the sterilization auxiliary.

### (Component (B): Acid)

The Component (B) is one or more acids selected from phosphoric acid, acetic acid, citric acid, malic acid, succinic acid, gluconic acid, lactic acid, and L-tartaric acid, and the Component (B) may be used either alone or as a combination of two or more.

If Component (B) is used, the occurrence of damage to a substance to be treated due to the pH of the sterilization auxiliary becoming too low may be suppressed. In addition, sufficient sterilization improving effect due to the Component (A) may be obtained since solubility of the aluminum ions is readily maintained.

Phosphoric acid and acetic acid have low reactivity with ozone, and therefore have an advantage of making it less likely to waste the ozone supplied in the sterilization auxiliary. In addition, by forming a chelating ligand with the aluminum ions, citric acid has an advantage of suppressing excess decomposition of the ozone by the aluminum ions, or suppressing the aluminum ions from becoming aluminum hydroxide.
Therefore, as the Component (B), one or more acid selected from the group consisting of phosphoric acid, citric acid, and acetic acid from the viewpoints of superior balance between molecular weight, coordination with the aluminum ions, and reactivity with ozone, and acetic acid is particularly preferable from the viewpoint of obtaining a high sterilizing effect.

The content of the Component (B) in the sterilization auxiliary of the present invention is preferably 10 to 10,000 mg/L, more preferably 100 to 2,000 mg/L, and even more preferably 100 to 1,000 mg/L. If the content of the Component (B) is greater than or equal to 10 mg/L, it is easy to obtain a high sterilizing effect. If the content of the Component (B) is less than or equal to 10,000 mg/L, it is less likely to inhibit the effects of the aluminum ions, and excessive consumption of ozone due to the reaction with ozone is readily suppressed.

The pH of the sterilization auxiliary of the present invention is greater than or equal to 1.0 and less than 5.0.
By maintaining the pH at greater than or equal to 1.0 and less than 5.0, ozone sterilization in which the loads on the sterilization apparatus and a substance to be treated are reduced and may obtain a high sterilizing effect against bacteria at the same time.
That is, by maintaining the pH at less than 5.0, it is possible to suppress the aluminum ions present in the sterilization auxiliary from being made insoluble by becoming hydroxides, and a high sterilizing effect may be obtained since the Component (A) can sufficiently act on bacteria. In addition, the lower the pH, the higher the sterilizing effect since the ozone-containing air bubbles readily adsorb the bacteria along with the ozone-containing air bubbles in the sterilization auxiliary being more stabilized.
On the other hand, by maintaining the pH at 1.0 or more, material deterioration caused by the hydrolysis, corrosion or dissolution of metal, rubber, plastic, or the like is suppressed, therefore the load on the sterilization apparatus and a container to be sterilized made of these materials is reduced at the time of ozone sterilization. More preferably, by maintaining the pH at 3.0 or more, the load on the sterilization apparatus and a container to be sterilized made of metal, rubber, plastic, or the like, is further reduced at the time of ozone sterilization.

In addition, when food is sterilized, damage to the food material is reduced. Furthermore, in the sterilization auxiliary of the present invention, the pH is more preferably 3.0 or more when food is a target of sterilization.

In addition, the above pH means a pH value measured at 25°C using a hydrogen electrode, or the like. However, the utilization temperature of the sterilization auxiliary of the present invention is not limited to this temperature and provided the pH is within the above range when converted to the pH value indicated at 25°C, the sterilization auxiliary of the present invention is within the scope of the present invention when used at any temperature.
In the sterilization auxiliary of the present invention, a further pH adjustment is not required if a target pH is obtained by using the Component (B) only, however, if a target pH is not obtained by using the Component (B) only, the pH is adjusted to a target pH by adding an appropriate amount of a pH adjusting Component (E) such as hydrochloric acid and sodium hydroxide.

### (Component (C): Glycerin Fatty Acid Ester)

The sterilization auxiliary of the present invention, especially when used in an ozone aeration method, preferably includes a glycerin fatty acid ester as the Component (C) in which a fatty acid having 1 to 10 carbon atoms and glycerin are ester-bonded, in addition to the Component (A) and the Component (B). If the Component (C) is included, contact efficiency with a substance to be treated is increased since the ozone-containing air bubbles formed by the aeration of ozone can be miniaturized, and, therefore, the floating speed of the ozone-containing air bubbles is reduced. In addition, a sterilizing effect is improved since sufficient sterilization even to a fine portion is readily possible. Furthermore, if the Component (C) is used, the cost of the apparatus is low since air bubbles may be readily miniaturized using an ejector, an air diffusion pipe, or the like, without using a variety of micro-air bubble generators. In addition, an improvement in the washing property may also be expected since the wettability of a substance to be treated is improved by lowering the surface tension.
Furthermore, if the Component (C) is used in the sterilization auxiliary of the present invention, although it is under acidic conditions in which it is difficult for ozone to react with the Component (C), it is predicted that a trace of organic peroxide is formed by the reaction. As a result, it is considered that the sterilizing effect is further improved due to the sterilizing effect of the organic peroxide being added.

As the Component (C), when fresh food is ozone-treated, considering the remote possibility of residue, using a glycerin fatty acid ester that does not have restricted of use in food additives is preferable. In addition, in the sterilization using an ozone aeration method, it is preferable to use those which easily suppresses an overflow due to the air bubbles deposited on the liquid surface, and also easily suppresses a reduction in the sterilizing effect due to a decrease in mechanical force. For these reasons, as the Component (C), triacetin, diacetin, monoacetin or Monocaprylin is more preferable. Furthermore, in the sterilization using an ozone aeration method, triacetin or Monocaprylin is even more preferable from the viewpoint of the air bubbles being less likely to be deposited even when the amount of aeration is increased.

In particular, as the sterilization auxiliary used in an ozone aeration method, a combination of triacetin and Monocaprylin is particularly preferable as the Component (C) from the viewpoint of readily achieving a high sterilizing power even under conditions in which less Component (A) and ozone are used, and the conditions such that the treatment time is shorter. Although the amount by which triacetin lowers the surface tension is small, the rate at which triacetin reduces the dynamic surface tension is fast, and the air bubbles are easily broken to be minified. On the other hand, although the rate at which Monocaprylin reduces the dynamic surface tension is slow compared to that of triacetin, the rate of Monocaprylin is sufficient for the miniaturization of the ozone-containing air bubbles and the amount by which Monocaprylin lowers the surface tension (an absolute amount of the surface tension decrease until the equilibrium is reached) is greater than that of triacetin. This allows miniaturization of the ozone-containing air bubbles even at low concentrations. Therefore, sufficient miniaturization of ozone-containing air bubbles is possible with fewer agents due to the synergistic effect of triacetin and Monocaprylin. Triacetin has excellent water solubility compared to Monocaprylin, and is inexpensive, and therefore, compared to cases in which only Monocaprylin is used, cost reduction may further be obtained while maintaining the same sterilizing effect. In addition, since triacetin has a weaker bitter taste compared to Monocaprylin, a reduction in quality is readily suppressed if a substance to be treated is food.

When the Component (C) is used as the sterilization auxiliary of the present invention, the content of the Component (C) in the sterilization auxiliary is preferably 10 to 5,000 mg/L, and more preferably 10 to 100 mg/L. If the content of the Component (C) is 10 mg/L or more, the effect by the Component (C) is readily obtained. If the content of the Component (C) is 5,000 mg/L or less, concerns such as the bubbles being deposited on the liquid surface of the sterilization auxiliary during an ozone treatment of a substance to be treated, or the Component (C) remaining in a substance to be treated after a treatment are less likely.

In addition, the sterilization auxiliary of the present invention may contain a variety of surfactants, aromatics, enzymes, fluorescent agents, thickeners, dispersing agents, inorganic salts, alcohols, sugars, or the like as additional components in order to improve usability or stabilization, or granting functions to the sterilization auxiliary as long as it does not inhibit an ozone oxidation reaction.
The surfactant is not particularly limited and may be appropriately selected from known surfactants in the related art depending on the purpose. For example, following Components (D1) to (D4), and the like, may be included.
Component (D1): an anionic surfactant.
Component (D2): a nonionic surfactant.
Component (D3): an amphoteric surfactant.
Component (D4): a cationic surfactant.

Examples of the Component (D1) may include an alkylbenzene sulfonate, an alkyl sulfate, an alkylphenyl ether sulfate, a polyoxyethylene alkyl ether sulfate, an acyl amide alkyl sulfate, an alkyl phosphate, a polyoxyethylene alkyl ether carboxylic acid, a paraffin sulfonate, an α-olefin sulfonate, an α-sulfo carboxylic acid and water-soluble salts such as esters thereof, soaps, or the like.
Examples of the Component (D2) may include an ethoxylated nonion such as a polyoxyethylene alkyl ether or a polyoxyethylene alkylphenyl ether, a sugar-based active agent such as a propylene glycol fatty acid ester, a glycerin fatty acid ester (except those corresponding to Component (C)), a propylene glycol fatty acid ester, a sorbitan fatty acid ester, a polyoxyethylene sorbitan fatty acid ester, a glucoside ester, a sugar ester, a methyl glucoside ester, an ethyl glucoside ester or an alkyl polyglucoside, an amide-based active agent such as an alkyl amine oxide, an alkyl diethanolamide, a fatty acid N-alkyl glucamide or an alkylamine oxide, or the like.
Examples of the Component (D3) may include an aminocarboxylate salt such as an alkylcarboxy betaine, an alkyl sulfoxy betaine, an alkyl amidopropyl betaine or an alkyl alaninate, an imidazoline derivative, an alkyl amine oxide, or the like.
Examples of the Component (D4) may include an alkyl trimethyl ammonium salt, a dialkyl dimethyl ammonium salt, or the like.
The surfactant may be used either alone or as a combination of two or more.

The content of the surfactant in the sterilization auxiliary is preferably 0 to 10 mg/L and more preferably 0 to 5 mg/L. If the content of the surfactant is 10 mg/L or less, the occurrence of undesirable phenomena on the process such as bubbling caused by the ozone-containing air bubbles being deposited on the surface of water and overflow may be readily suppressed.

### (Preparation Method)

The sterilization auxiliary of the present invention is prepared by adding the Component (A), the Component (B), and other components, as necessary, to water, and adjusting the pH using hydrochloric acid or sodium hydroxide, if necessary.
The water used in the sterilization auxiliary is not particularly limited. Ozone reacts with dissolved metals, chlorine, organic substances or the like due to its strong oxidizing power, therefore water having few of these impurities, and having high purity is preferable. However, water may be appropriately selected depending on the type of substance to be treated and the degree of sterilization required, and tap water may be used.

### [Ozone Sterilization Method]

An ozone sterilization method of the present invention is a method in which a substance to be treated is ozone-treated using the sterilization auxiliary for ozone sterilization of the present invention described above. Ozone has been known to have a high sterilizing power due to its strong oxidizing power. The ozone sterilization method of the present invention may employ well-known ozone sterilization methods except that the sterilization auxiliary of the present invention is used. The ozone sterilization method of the present invention may be any of the following method (α) and the method (β).
(α) an ozone aeration method in which a substance to be treated is immersed in the sterilization auxiliary of the present invention, and the substance to be treated is ozone-treated by aerating ozone-containing gas in the above sterilization auxiliary.
(β) an ozone water immersion method in which a substance to be treated is immersed in the sterilization auxiliary composition in which ozone is dissolved in the sterilization auxiliary, and the substance to be treated is ozone-treated by using ozone water.

The method (β) has a tendency for a waste of ozone compared to the method (α) since ozone-containing gas which could not be dissolved is aerated. In addition, the amount of the ozone water used tends to be large as the dissolved ozone reacts with impurities since it has a faster reaction rate and has no selectivity in the reaction, and also has high permeability to food materials if the substance to be treated is food. Furthermore, if the substance to be treated is food, there is also a concern that the quality of food materials is deteriorated after the ozone treatment since the dissolved ozone penetrates food during sterilization. From these points, as the ozone sterilization method of the present invention, the method (α) is preferable to the method (β).

### (Method (α))

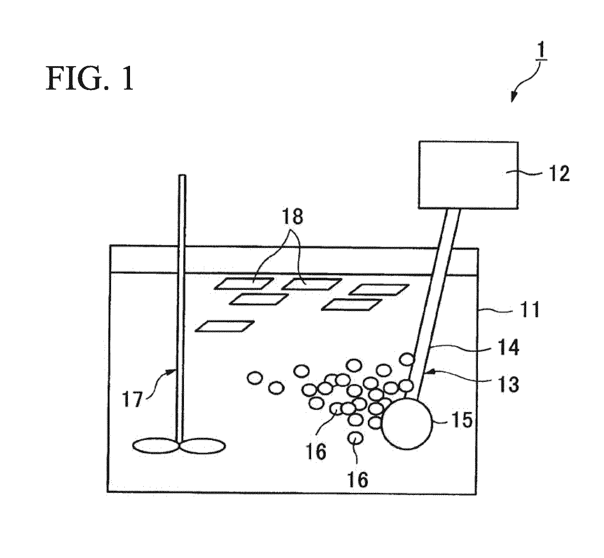

The method (α) is a method having a step in which a substance to be treated is immersed in the sterilization auxiliary of the present invention described above, and ozone-containing gas is aerated in the above sterilization auxiliary in which the substance to be treated is immersed. Hereinafter, one example of the embodiments of the method (α) will be described with reference to FIG. 1. FIG. 1 is a schematic diagram showing one example of sterilization apparatus used in the method (α).
The sterilization apparatus 1, as shown in FIG. 1, includes a water tank 11, and ozone-containing gas supply means 12, aeration means 13, and stirring means 17. The aeration means 13 is configured of a supply pipe 14, and an air diffusion unit 15 provided at the distal end of the supply pipe 14. The air diffusion unit 15 is immersed in the sterilization auxiliary stored in the water tank 11, and the supply pipe 14 is connected to the ozone-containing gas supply means 12. The stirring means 17 is provided within the water tank 11.

The materials of the water tank 11 are not particularly limited, however, are preferably materials having excellent resistance to the strong oxidizing power of ozone, and the use of glass, Teflon (Registered trademark) (polytetrafluoroethylene), titanium, aluminum or stainless steel treated with ozone, that is, in which a strong oxide film is formed by a high concentration of ozone is preferable. A water tank made of materials such as nitrile rubber or urethane which has low resistance to ozone may be used, however, in such a case, sufficient care regarding deterioration of the water tank 11 must be taken.
The size of the water tank 11 may be determined considering the amount of a substance to be treated which is ozone-treated, and the performance of the stirring means 17.

The ozone-containing gas supply means 12 may be any of those which can supply ozone-containing gas containing ozone and, for example, an ozone generator, a cylinder filled with ozone-containing gas may be included. In addition, an apparatus, which generates ozone using an ozone generator, sends the ozone generated to a mass flow controller through a regulator, and supplies ozone in the sterilization auxiliary while controlling the flow rate with the mass flow controller, may be used.
The ozone generator is not particularly limited and, for example, those using methods in which light of high energy such as an electron beam, radiation or ultraviolet rays is irradiated on oxygen, chemical methods, electrolysis, discharge methods, or the like, may be included. In industry, those using a silent discharge method are often used in terms of the cost and amount of ozone-containing gas generated. Example of such commercially available ozone generators may include YGR-50 which is a low concentration ozone generator (trade name, manufactured by Iwaki & Co., Ltd.), ED-OG-R4 which is a high concentration ozone generator (trade name, manufactured by Ecodesign, Inc.), or the like.

The aeration means 13 may be any of those which can supply ozone-containing air bubbles in the sterilization auxiliary by aerating the ozone-containing gas and may employ, for example, a well-known apparatus such as a diffusion plate, a diffusion container, a diffuser or an ejector. By using such apparatus, the sterilizing effect of a substance to be treated may be increased by generating as fine ozone-containing air bubbles as possible.

The stirring means 17 may be any of those which can stir the sterilization auxiliary within the water tank 11, and may be those using a stirring blade or those generating water flow using a pump, or the like.

Hereinafter, one example of the method (α) using the sterilization apparatus 1 will be described. As the method (α), for example, a method having the following steps may be included.
Pre-washing step: a step in which a substance to be treated is washed with water prior to ozone treatment.
Ozone aeration treatment step: a step in which the substance to be treated is immersed in the sterilization auxiliary which is stored in the water tank 11 of the sterilization apparatus 1, and the substance to be treated is ozone-treated by aerating ozone-containing gas in the above sterilization auxiliary.
Rinsing step: a step in which the substance to be treated is rinsed with water after sterilization, and the sterilization auxiliary is washed off.
Dewatering step: a step in which the substance to be treated is dewatered.
However, the method (α) is not limited to the above method as long as the above ozone aeration treatment step is included.

### Pre-washing Step:

The substance to be treated which is sterilized and cleaned was pre-washed using tap water, or the like and dirt, or the like is cleaned off. If the substance to be treated is food, the pre-washing step is not to be performed excessively, particularly so that the appearance is not deteriorated due to physical damage, and the quality is not reduced by water-soluble components such as vitamin C being eluted.

### Ozone Aeration Treatment Step:

First, the substance to be treated 18 which is to be sterilized is immersed in the sterilization auxiliary by placing an arbitrary amount of the sterilization auxiliary in the water tank 11 of the sterilization apparatus 1. Next, the ozone-containing air bubbles 16 are generated in the sterilization auxiliary by circulating the ozone-containing gas from the ozone-containing gas supply means 12 to the supply pipe 14, and aerating ozone-containing gas from the air diffusion unit 15. In the method (α), it is preferable that the ozone-containing air bubbles 16 be supplied as fine air bubbles using the sterilization auxiliary including the above Component (C) from the viewpoint of improving the sterilizing effect. Fine air bubbles means air bubbles with an average air bubble diameter of 500 µm or less. The average air bubble diameter of the ozone-containing air bubbles 16 is preferably 1 to 100 µm. The average air bubble diameter of the ozone-containing air bubbles 16 is measured by image analysis using a digital scope or a digital camera.

The sterilization of the substance to be treated 18 is carried out for an arbitrary period of time while generating the ozone-containing air bubbles 16 by stirring the sterilization auxiliary within the water tank 11 using the stirring means 17. In the sterilization auxiliary, the substance to be treated 18 is believed to be sterilized by the aluminum ions generated by the Component (A) being dissolved and the ozone-containing air bubbles 16 acting on bacterial cells. Furthermore, if the sterilization auxiliary including the above Component (C) is used, in the sterilization auxiliary, an organic peroxide is produced by some of the ozone supplied from the air diffusion unit 15 being dissolved in water and reacted with the Components (C), and it is believed that the above organic peroxide also contributes to the sterilization of the substance to be treated.

The substance to be treated 18 may be any of those in which a general ozone treatment is carried out, and fresh vegetables such as cut vegetables; kitchenware such as kitchen knives, cutting boards, dishes or sponges; toilet articles such as toilet seats; bath supplies such as a bucket or a bathtub; textile products such as clothes, sheets or blankets; medical devices such as endoscopes or scalpels; fresh food such as fruit, meat, fish, shellfish or eggs, and processed foods thereof; body parts such as fingers or an oral cavity; equipment such as production lines of factories, packaging containers, walls, floors or plumbing; sludge, or the like, may be included.

As the ozone-containing gas, ozone generated from an ozone generator may be used as is, and ozone diluted with diluent gas may also be used. As the diluent gas, a gas which is inert or has poor reactivity with ozone is preferable. Examples of the diluent gas include helium, argon, carbon dioxide, oxygen, air, nitrogen, or the like. Ozone is preferably used immediately after being prepared from an ozone generator since it has an autolyzing property.

The ozone concentration in the ozone-containing air bubbles (ozone-containing gas) is preferably 0.0005 to 1.0% by volume and more preferably 0.005 to 1.0% by volume. If the above ozone concentration is 0.0005% by volume or more, a high sterilizing power is readily obtained. If the above ozone concentration is 1.0% by volume or less, the concentration of ozone in the working environment is difficult to exceed the reference value, and deterioration in the quality of the substance to be treated after an ozone treatment is readily suppressed.
The amount of the ozone-containing gas supplied to the sterilization auxiliary may be determined depending on the purpose of sterilization, the type and the amount of the substance to be treated.

The time taken to aerate the ozone-containing gas to the sterilization auxiliary (aeration time) may be determined considering the degree of sterilization required, the type and the amount of the substance to be treated 18 in the sterilization auxiliary and the temperature of the auxiliary sterilization, and is preferably 1 to 10 minutes. If the time is within the above range, an adverse impact on the substance to be treated 18 becomes very small.
The temperature of the sterilization auxiliary during the aeration of the ozone-containing gas (aeration temperature) may be determined considering the degree of sterilization required, the types and the amount of the substance to be treated 18 in the sterilization auxiliary and the aeration time. The temperature of the sterilization auxiliary is preferably 0 to 50°C from the viewpoint of the ozone in the sterilization auxiliary becoming relatively stable. If the substance to be treated is food, the temperature of the sterilization auxiliary is more preferably 0 to 30°C.

### Rinsing Step:

The sterilization auxiliary adhered to the substance to be treated 18 is removed by rinsing with tap water, or the like. As the method of rinsing is not particularly limited and, for example, a method in which the substance to be treated is immersed in tap water which is being stirred, or the like, may be included. The number of rinses and the rinsing time is not made to be excessive considering cost or deterioration of appearance due to physical damage caused by stirring during rinsing or the like, and reduction of the quality by water-soluble components such as vitamin C being eluted if the substance to be treated is the food.

### Dewatering Step:

The substance to be treated 18 is dewatered after rinsing. The dewatering method is not particularly limited and, for example, a method in which dewatering is carried out using a dewatering machine which uses centrifugal force such as a dewatering tub of a washing machine.

### (Method (β))

The Method (β) is a sterilization method using ozone water, and has a step in which ozone is dissolved in the sterilization auxiliary and made to be a sterilizing agent composition, and the substance to be treated is ozone-treated by being immersed in the above sterilizing agent composition. The Method (β) is not particularly limited as long as the method has a step of ozone treatment using the above sterilizing agent composition and, for example, a method having the following steps, or the like, may be included.
Pre-washing step: a step in which a substance to be treated is washed with water prior to an ozone treatment in advance.
Ozone water immersion treatment step: a step in which the sterilizing agent composition in which ozone is dissolved in the sterilization auxiliary is put into the water tank of the sterilization apparatus, and the substance to be treated is ozone-treated by immersing the substance to be treated in the above sterilizing agent composition.
Rinsing step: a step in which the substance to be treated is rinsed with water after sterilization, and the sterilization auxiliary is washed off.
Dewatering step: a step in which the substance to be treated is dewatered.
However, the method (β) is not limited to the above method as long as the above ozone water immersion treatment step is included.

The pre-washing step, rinsing step and the dewatering step in the method (β) are the same as the pre-washing step, rinsing step and the dewatering step described in the method (α).

### Ozone Water Immersion Step:

The preparation method of the above sterilizing agent composition is not particularly limited and include, for example, a method in which an aqueous solution with a pH of greater than or equal to 1.0 and less than 5.0 including the Component (A), the Component (B), and other components are mixed as necessary with ozone water prepared in advance. The preparation method of the above ozone water is not particularly limited and may include a method in which ozone is generated in water, a method in which ozone gas generated once out of water is dissolved in water, or the like.
As the method for generating ozone in water, a method using electrolysis of water is most common.
As the method for dissolving ozone gas in water, a method in which ozone gas is generated from the ozone generator described above or the like and the above ozone gas is aerated into water, a method using a diffuser, a method in which ozone gas is dissolved in water through a film made of Teflon (registered trademark) or the like, or the like, may be included.

The content of ozone in the above sterilizing agent composition in which ozone is dissolved is preferably 0.01 to 5 mg/L and more preferably 0.1 to 5 mg/L. If the content of ozone in the above sterilizing agent composition is 0.01 mg/L or more, a high sterilizing effect is readily obtained. If the content of ozone in the above sterilizing agent composition is 5 mg/L or less, a deterioration in quality of the substance to be treated after the ozone treatment is readily suppressed.

The method (β) may be performed using sterilization apparatus having at least a water tank which stores the above sterilizing agent composition, and a well-known apparatus may be used. The materials of the water tank used in the method (β) include the same materials included as the materials of the water tank 11 in the sterilization apparatus 1, and preferable aspects are the same.

According to the ozone sterilization method which uses the sterilization auxiliary of the present invention described above, a higher sterilizing effect may be obtained with a smaller amount of ozone since the sterilizing power is improved by including the Component (A) in the sterilization auxiliary. Therefore, it is not necessary to use additional facilities to generate high concentration ozone or to reduce the ozone concentration in the atmosphere of a working environment, and the cost can be reduced. In addition, the load on the sterilization apparatus may be reduced since the amount of ozone used can be reduced.

Although the cause of the effect of improving the sterilizing power using the Component (A) is not clear, it is considered to be as follows.
Aluminum ions are trivalent cations and are known to denature protein by binding to the protein. Utilizing this property, aluminum has been used, for example, to prevent the breakdown of sea urchin meat, and as an astringent in antiperspirant. When the sterilization auxiliary of the present invention is used, it is postulated that bacterial cells are readily killed since the Component (A) decreases the activity of the bacterial cells by acting on the membrane protein of the bacterial cells, and ozone performs oxidative decomposition on the above bacterial cells. In addition, it is known that metal ions catalyze the decomposition of ozone and generate hydroxyl radicals which have a stronger oxidizing power and it is postulated that this also contributes to the improved sterilizing power of the Component (A).

### [Examples]

Hereinafter, the present invention will be described in detail with examples and comparative examples. However, the present invention is not limited by the following description.

### <Raw Materials Used>

Hereinafter, raw materials used in the present examples are shown.

### (Component (A))

Component A11: aluminum potassium sulfate (manufactured by Taimei Chemicals Co., Ltd., dry matter for food additives, AlK(SO₄)₂· 12H₂O)
Component A12: ammonium aluminum sulfate (manufactured by Taimei Chemicals Co., Ltd., dry matter for food additives, AlNH₄(SO₄)₂-12H₂O)
Component A13: burnt potassium alum (AlK(SO₄)₂)
Component A14: burnt ammonium alum (AlNH₄(SO₄)₂)
Component A2: aluminum chloride (manufactured by Kanto Chemical Co., Inc.)

### (Component (A'): Comparison Component of Component (A))

Component A' 1: iron sulfate heptahydrate (manufactured by Kanto Chemical Co., Inc., a food additive standardized product)
Component A'2: copper sulfate pentahydrate (manufactured by Kanto Chemical Co., Inc., a food additive standardized product)

### (Component (B))

Component B1: phosphoric acid (manufactured by Junsei Chemical Co., Ltd., a food additive standardized product)
Component B2: citric acid (manufactured by Junsei Chemical Co., Ltd., a food additive standardized product)
Component B3: acetic acid (glacial acetic acid) (manufactured by Junsei Chemical Co., Ltd., a food additive standardized product)
Component B4: malic acid (manufactured by Junsei Chemical Co., Ltd., a food additive standardized product)
Component B5: succinic acid (manufactured by Junsei Chemical Co., Ltd., a food additive standardized product)
Component B6: gluconic acid (manufactured by Kanto Chemical Co., Inc.)
Component B7: lactic acid (manufactured by Junsei Chemical Co., Ltd., a food additive standardized product)
Component B8: L-tartaric acid (manufactured by Kanto Chemical Co., Inc.)

### (Component (B'): Comparison Component of Component (B))

Component B'1: ascorbic acid (manufactured by Junsei Chemical Co., Ltd., a food additive standardized product)

### (Component (C))

Component C1: Monocaprylin (manufactured by Taiyo Kagaku Co., Ltd., Sunsoft No. 700P-2)
Component C2: triacetin (manufactured by Kanto Chemical Co., Inc.)

### (Component (E): pH Adjusting Agent)

Component E1: 0.1 N aqueous solution of hydrochloric acid (manufactured by Kanto Chemical Co., Inc.)
Component E2: 0.1 N aqueous solution of sodium hydroxide (manufactured by Kanto Chemical Co., Inc.)

### <Potential Sterilization Test>

For the sterilization auxiliary of the present invention, a potential sterilization test was performed on microorganism dispersed in an aqueous solution in order to confirm the presence or absence of a basic sterilizing effect with respect to microorganisms.

### [Examples 1 to 12]

Escherichia coli (NBRC3972 strain) was cultured for 24 hours at 37°C (hereinafter, also referred to as "pre-pre-culture") in an SCD agar medium (manufactured by Nissui Pharmaceutical Co., Ltd.), the cultured Escherichia coli was further cultured in the same manner as the pre-pre-culture (hereinafter, also referred to as "pre-culture"), and the pre-cultured Escherichia coli was used in a sterilization test.
The pre-cultured Escherichia coli was dispersed in a buffer liquid containing a peptone (a liquid in which 3.56 g of potassium dihydrogenphosphate, 18.2 g of disodium dihydrogenphosphate dodecahydrate, 4.3 g of sodium chloride and 1.0 g of peptone were dissolved in 1 L of purified water, and were neutralized to pH 7.0), and a bacterial liquid with a number of bacteria in the vicinity of 1.0×10⁸ cfu/mL was prepared using the transmittance of light at a wavelength of 660 nm as an indicator.

Pure water was introduced to a test tube made of glass, and each component was added so that the amounts in the sterilization auxiliary reached the values shown in Table 1, the pH was adjusted, BSA (bovine serum albumin) manufactured by SIGMA-Aldrich Co. LLC.was further added as a contaminant to become 0.01 % by mass, and in total, 10 mL of the sterilization auxiliary was prepared. 100 µL of the bacterial liquid was added to the sterilization auxiliary immediately before aerating the ozone-containing gas and a sample liquid in which the initial number of bacteria was set at 1.0×10⁵ cfu/mL was prepared.

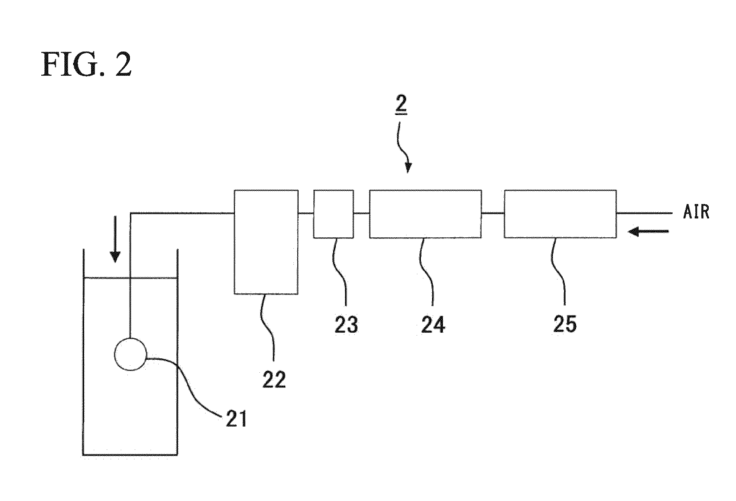

The ozone aeration means 2 exemplified in FIG. 2 was used in the sterilization test of the sample liquid. In the ozone aeration means 2, an air diffusion unit 21 (Kinoshita-type glass filter 501G (No.4), manufactured by Kinoshitarika), an ozone concentration meter 22 (with a built-in pump, PG-620MA, manufactured by Ebara Jitsugyo Co., Ltd.), a mass flow controller 23 (MODEL8500, manufactured by Kojima Instruments Inc.), an ozone generator 24 (Aqua Zone 200, manufactured by Red Sea Fish Pharm Ltd.), and a dehumidification unit 25 (silica gel, 500 mL) are connected in this order. In the ozone aeration means 2, ozone aeration is carried out by dehumidifying the air using the dehumidification unit 25 and supplying dry air to the ozone generator 24, generating ozone-containing gas from the ozone generator 24 and supplying the ozone-containing gas from the air diffusion unit 21 while controlling the flow rate using the mass flow controller 23. Ozone concentration in the ozone-containing gas may be measured using the ozone concentration meter 22. Ozone-containing gas with an ozone concentration of 0.01% by volume (diluent gas: air) was aerated for 2 minutes into the above sample liquid in a test tube at a flow rate of 20 mL/minute using ozone aeration means 2. The treatment temperature was set at 25°C.

### [Comparative Examples 1 to 11]

Each component was added so that the amount in the sterilization auxiliary reached the values shown in Table 1, the pH was adjusted, and the ozone aeration treatment was performed in the same manner as that of Examples 1 to 12.
In addition, the pH of the sterilization auxiliary was adjusted by adding an appropriate amount of 0.1 N aqueous solution of hydrochloric acid solution (Component E1) or 0.1 N aqueous solution of sodium hydroxide (Component E2) as necessary. A pH meter (Seven Easy, manufactured by METTLER TOLEDO) was used to measure the pH.

### [Evaluation Method]

In each example, after the ozone aeration treatment, 5 mL of the sample liquid was immediately collected in a separate test tube sterilized in advance and was diluted stepwise by 10 times, respectively, using a buffer liquid containing peptone. For the diluted liquid of each stage, after 1 mL of each was collected using a micropipette and added dropwise to a petri dish, each was mixed with 10 mL of SCD agar medium (manufactured by Nissui Pharmaceutical Co., Ltd.) which was kept warm at approximately 50°C. Next, solidification of the agar was confirmed, the diluted liquid of each stage was cultured on two petri dishes under the conditions of 36°C and 24 hours using an incubator. After that, the number of residual bacteria (number of bacteria) was examined by counting the number of colonies on the medium for the cultured diluted liquid of each stage with the number of colonies being 300 cfu or less per petri dish. The number of residual bacteria is a value obtained by averaging the number of bacteria which was counted for the two petri dishes. In addition, the number of bacteria measured in the same manner as above for the sample liquid prior to the ozone aeration treatment was made to be the number of initial bacteria, and sterilizing power was evaluated as -log(number of residual bacteria/number of initial bacteria).
The evaluation of the sterilizing power is "0" (-log(1.0×10⁵/1.0×10⁵)=0) if the number of bacteria is not reduced in the present test since the number of initial bacteria is set at 1.×10⁵ cfu/mL in the present test. On the other hand, the evaluation of the sterilizing power is "5" (-log(1.0×10⁰/10×10⁵)=5) if the bacteria were wiped out by the ozone aeration treatment. In the present test, it was determined as a pass if the evaluation result was "3.0" or more indicating that the number of bacteria had been reduced to 1/1000 from the number of initial bacteria since such a result is practicable.
Evaluation results of the sterilizing power in Examples 1 to 12 and Comparative Examples 1 to 11 are shown Table 1.

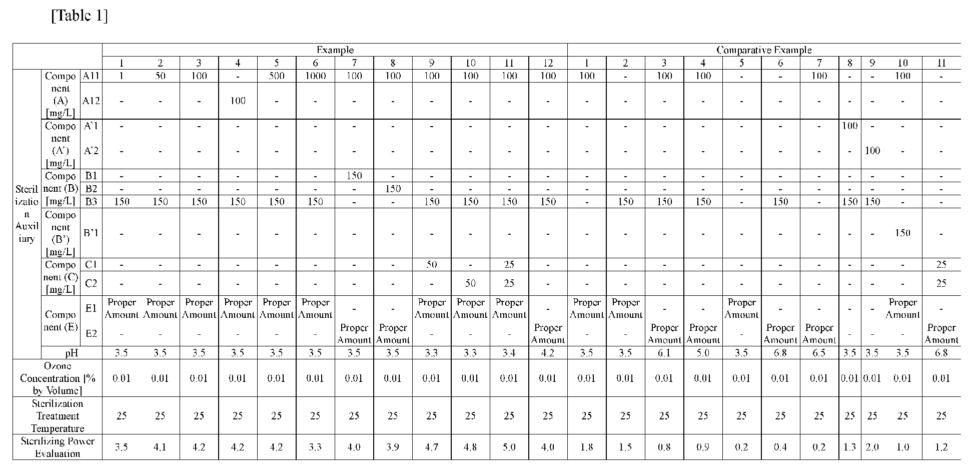

As shown in Table 1, in Examples 1 to 12 which used the sterilization auxiliary which included the Component (A) and the Component (B), a high sterilizing effect was obtained even with a treatment for a short period of time with a small amount of ozone. When Examples 3 and 7 and 8, which used the sterilization auxiliary including different types of the Component (B) in the same amount, were compared, in a case in which acetic acid was used as the Component (B) (Example 3), a higher sterilizing effect was obtained. In addition, in Examples 9 to 11 which used the sterilization auxiliary which included the Component (C) in addition to the Component (A) and the Component (B), a higher sterilizing effect was obtained by the ozone-containing air bubbles being miniaturized. In particular, in Example 11 using a combination of Monocaprylin and triacetin as the Component (C), a sterilizing effect higher than that of a case using either Monocaprylin or triacetin alone was obtained.

On the other hand, in Comparative Example 1 which used the sterilization auxiliary which did not include the Component (B), in Comparative Example 2 which used the sterilization auxiliary which did not include the Component (A), and in Comparative Examples 3 and 4 which used the sterilization auxiliary which included the Component (A) and the Component (B), however, had a pH of 5.0 or more, the sterilizing power was smaller compared to that of the examples.
In addition, in Comparative Examples 5 to 7 which used the sterilization auxiliary in which two of the three conditions of the Component (A), the Component (B) and the pH being greater than or equal to 1.0 and less than 5.0 were not satisfied, sterilizing power became extremely small.
Furthermore, in Comparative Examples 8 and 9 which used other metal salts of iron or copper instead of the aluminum salts of the Component (A), the sterilizing power was smaller than that of examples and the effect of improving the sterilizing effect was not obtained.
In addition, In Comparative Example 10 which used L-ascorbic acid instead of the acid of the Component (B), the sterilizing power was smaller than that of examples and the effect of improving the sterilizing effect was not obtained.
Furthermore, in Comparative Example 11 in which the ozone-containing air bubbles were miniaturized by adding triacetin and Monocaprylin of the Component (C), sterilizing power was smaller.

### <Solid Surface Sterilization Test>

### [Examples 13 to 32]

Escherichia coli (NBRC3972 strain) was cultured for 24 hours at 37°C (hereinafter, also referred to as "pre-pre-culture") in an SCD agar medium (manufactured by Nissui Pharmaceutical Co., Ltd.), the cultured Escherichia coli was further cultured in the same manner as the pre-pre-culture (hereinafter, also referred to as "pre-culture"), and the pre-cultured Escherichia coli was used in a sterilization test. The pre-cultured Escherichia coli was diluted with sterile water and a bacterial diluent with a number of bacteria in the vicinity of 2.0×10⁷ cfu/mL was prepared using the light transmittance of light at a wavelength of 660 nm as an indicator. A bacterial liquid in the vicinity of 1.0×10⁷ cfu/mL was prepared by dispersing the above bacterial diluent in into a nutrient broth at a ratio of 5:5 (manufactured by Kanto Chemical Co., Inc.).
Next, 10 µL of the above bacterial liquid was respectively coated on 2 cmx2 cm SUS304 (hairline finishing product), silicon rubber (manufactured by Tigers Polymer Co., Ltd.) and surface polished glass (polished by a waterproof abrasive paper No. 1200 manufactured by Sankyo-Rikagaku Co., Ltd.), was natural dried for 30 minutes, and was made to be the test piece for a solid surface sterilization test.

In addition, each component was added to pure water so that the amount in the sterilization auxiliary reached the values shown in Table 2, BSA (bovine serum albumin) manufactured by SIGMA-Aldrich Co. LLC. was further added as a contaminant to become 0.01% by mass, and the sterilization auxiliary was prepared.

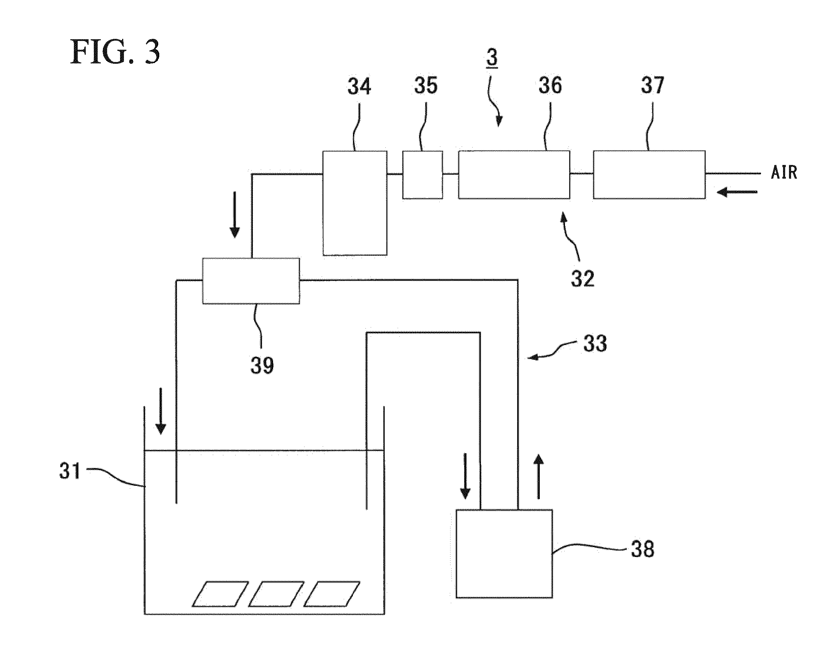

In the sterilization test of the above test pieces, self-manufactured sterilization apparatus 3 exemplified in FIG. 3 was used.
The sterilization apparatus 3 is equipped with a water tank 31 (a 3 L polypropylene beaker), ozone-containing gas supply means 32, and air bubble generation means 33 as shown in FIG. 3. In the ozone-containing gas supply means 32, an ozone concentration meter 34 (with a built-in pump, PG-620MA, manufactured by Ebara Jitsugyo Co., Ltd.), a mass flow controller 35 (MODEL8500, manufactured by Kojima Instruments Inc.), an ozone generator 36 (Aqua Zone 200, manufactured by Red Sea Fish Pharm Ltd.), and a dehumidification unit 37 (silica gel, 500 mL) are connected in this order. The air bubble generation means 33 has a volute pump 38 (manufactured by Elepon E.C.A.P. Corporation, SL-5SN) and an ejector 39 (manufactured by As One Corporation, an aspirator made of polytetrafluoroethylene (PTFE)), and the inside of the water tank 31 and the volute pump 38, the volute pump 38 and the ejector 39, and the ejector 39 and the inside of the water tank 31 are connected by piping (made of vinyl chloride, joint: SUS304). The ozone concentration meter 34 of the ozone-containing gas supply means 32 and the ejector 39 of the air bubble generation means 33 are also connected. The piping with an inner diameter of 6 mm is connected to the inlet of the sterilization auxiliary (volute pump 38 side), the piping with an inner diameter of 4 mm is connected to the inlet of the ozone-containing gas (the ozone concentration meter 34 side), and the piping with an inner diameter of 6 mm is connected to the outlet of the sterilization auxiliary (the water tank 31 side).
In the sterilization apparatus 3, ozone-containing gas is supplied to the ejector 39 by dehumidifying the air using the dehumidification unit 37 and supplying dry air to the ozone generator 36, generating the ozone-containing gas from the ozone generator 36 and controlling the flow rate using the mass flow controller 35. The ozone concentration in the ozone-containing gas may be measured using the ozone concentration meter 34. In addition, the volute pump 38 supplies the sterilization auxiliary in the water tank 31 by the rotation of a waterwheel having a radial-shaped groove on the outer periphery (not shown), the ozone-containing gas and the sterilization auxiliary are gas-liquid mixed in the ejector 39 generating the ozone-containing air bubbles, and the ozone-containing air bubbles are supplied into the water tank 31.
The sterilization auxiliary (1400 mL) was stored inside the water tank 31 of the sterilization apparatus 3, three of the test pieces were provided at the bottom of the water tank 31 with the bacteria adhered surface facing up, in positions which are not directly exposed to the water flow from the ejector 39. Then, the sterilization auxiliary was circulated at 3 L/minute using the volute pump 38, ozone-containing gas with the ozone concentration of 0.01% by volume (that is, mixed gas of diluent gas and air) was supplied from the ozone generator 36 to the ejector 39 at a flow rate of 20 mL/minute generating ozone-containing air bubbles, and the ozone aeration treatment was performed by supplying the ozone-containing air bubbles to the sterilization auxiliary in the water tank 31 for 5 minutes. The treatment temperature was set at 25°C.

### [Comparative Examples 12 to 21]

Ozone aeration treatment was performed for each test piece in the same manner as that of Examples 13 to 32, except that the addition amount of each component of the sterilization auxiliary used and the pH were changed as shown in Table 3.
In addition, the pH of the sterilization auxiliary was adjusted by adding an appropriate amount of 0.1 N aqueous solution of hydrochloric acid solution (Component E1) or 0.1 N aqueous solution of sodium hydroxide (Component E2) as necessary. A pH meter (Seven Easy, manufactured by METTLER TOLEDO) was used to measure the pH.

### [Evaluation Method of Solid Surface Sterilizing Effect]

In each example, after the ozone aeration treatment, the test piece was taken out using sterile tweezers, and bacteria were collected in 10 mL of sterile water from the surface of the test piece according to a swabbing method (a method in which bacteria are collected from the surface using a cotton swab wet with sterile water) using Fukifuki Check II (manufactured by Eiken Chemical Co., Ltd.). This liquid was collected in a test tube sterilized in advance and was diluted stepwise by 10 times, respectively, using a buffer liquid containing peptone. For the diluted liquid of each stage, after 1 mL of each was collected using a micropipette and was added dropwise a petri dish, each was mixed with a SCD agar medium (manufactured by Nissui Pharmaceutical Co., Ltd.) which was kept warm at approximately 50°C. Next, solidification of the agar was confirmed, the diluted liquid of each stage was cultured on two petri dishes under the conditions of 36°C and 24 hours using an incubator. After that, the number of residual bacteria (number of bacteria) was examined by counting the number of colonies on the medium for the cultured diluted liquid of each stage with the number of colonies being 300 cfu or less per petri dish. The number of residual bacteria is a value obtained by averaging the number of bacteria which was counted for the two petri dishes. In addition, the number of bacteria measured in the same manner as above for the sample liquid prior to the ozone aeration treatment was made to be the number of initial bacteria, and sterilizing power was evaluated as -log(number of residual bacteria/number of initial bacteria).
The evaluation of the sterilizing power is "0" if the number of bacteria is not reduced in the present test. On the other hand, the evaluation of the sterilizing power is "4" if the bacteria were wiped out by the ozone aeration treatment. This means that, in the above evaluation method, a certain number of the bacteria were measured as a background even if the bacteria on the surface of the test piece were wiped out. Furthermore, in the present test, the number of bacteria is reduced only by air aeration depending on the water flow and the number of bacteria can be reduced to 1/10 or less although it varies depending on the material of the test piece. In the present test, it was determined as a pass if the number of bacteria was reduced to 1/100 or less with respect to the initial number of bacteria, that is, -log(number of residual bacteria/number of initial bacteria) is "2.0" or more since such a result is practicable.

### [Evaluation Method of Solid Surface Deterioration]

After the bacteria were collected using the swabbing method, the test piece was placed at the bottom of the water tank 31 again, the sterilization auxiliary was circulated at 3 L/minute using the volute pump 38, ozone-containing gas with an ozone concentration of 0.01% by volume (diluent gas: air) was supplied from the ozone generator 36 to the ejector 39 at a flow rate of 20 mL/minute generating ozone-containing air bubbles, and the ozone aeration treatment was performed by supplying the ozone-containing air bubbles to the sterilization auxiliary in the water tank 31 for 6 hours.
After the ozone treatment, the test piece was taken out and the appearance thereof was evaluated visually. The evaluation criteria are shown below. In addition, in the appearance evaluation, 3 points or more was determined as O (pass) since it is practicable.

### Surface Polished Glass

4 points: No change compared to pretreatment.
3 points: Little change compared to pretreatment.
2 points: Compared to pretreatment, soot has appeared and it was somewhat difficult to see the opposite side when the test piece was emptied.
1 point: Compared to pretreatment, soot has appeared and it is quite difficult to see the opposite side when the test piece was emptied.

### SUS304

4 points: No change compared to pretreatment.
3 points: Little change compared to pretreatment.
2 points: Slight corrosion compared to pretreatment.
1 point: Compared to pretreatment, the ratio of the corroded portion was 20% or more.

### Silicon rubber

4 points: No changes compared to pretreatment.
3 points: Little changes compared to pretreatment.
2 points: Slight discoloration compared to pretreatment.
1 point: Compared to pretreatment, discoloration was observed and the ratio of the cracked portion was 20% or more.

### [Comprehensive Evaluation]

In the present test, it was comprehensively determined as O (pass) if the results obtained from the solid surface sterilization test were all 2.0 or more, and the results obtained from the solid surface deterioration test were all 3.0 or more.
In addition, if there were results in which the score did not reach to the above criteria in any one of the solid surface sterilization test or the solid surface deterioration test, it was determined as X (fail).

For the solid surface sterilization test and the solid surface deterioration test, the results of Examples 13 to 32 are shown in Table 2, and the results of Comparative Examples 12 to 21 are shown in Table 3.

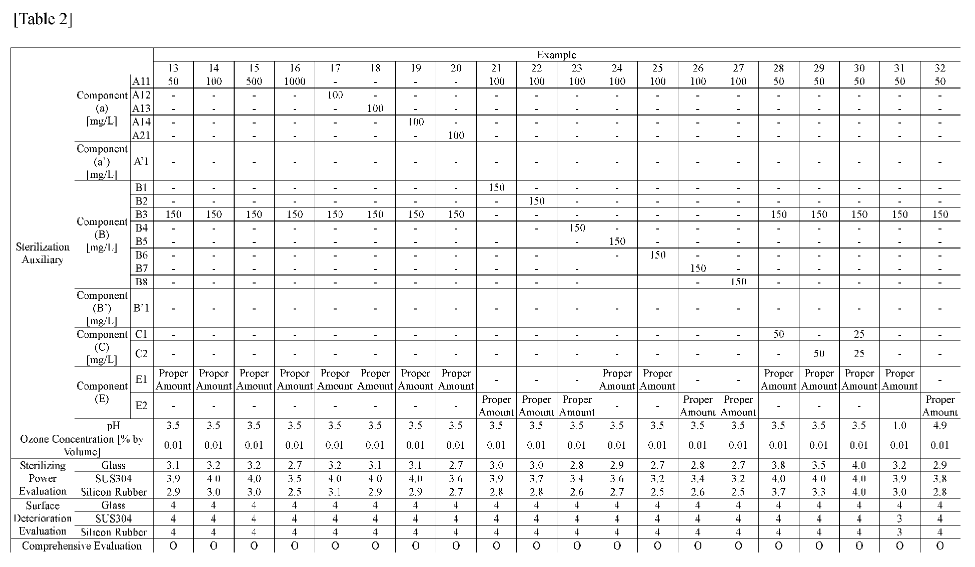

**[Table 3]**

| | | | Comparative Example | | | | | | | | | |
|---|---|---|---|---|---|---|---|---|---|---|---|---|
| | | | 12 | 13 | 14 | 15 | 16 | 17 | 18 | 19 | 20 | 21 |
| Sterilization Auxiliary | Component (a) [mg/L] | A11 | 50 | - | 50 | 50 | 50 | 50 | - | - | - | 50 |
| | | A12 | - | - | - | - | - | - | - | - | - | - |
| | | A13 | - | - | - | - | - | - | - | - | - | - |
| | | A14 | - | - | - | - | - | - | - | - | - | - |
| | | A21 | - | - | - | - | - | - | - | - | - | - |
| | Component (a') [mg/L] | A'1 | - | - | - | - | - | - | - | - | 50 | - |
| | Component (B) [mg/L] | B1 | - | - | - | - | - | - | - | - | - | - |
| | | B2 | - | - | - | - | - | - | - | - | - | - |
| | | B3 | - | 150 | 150 | 150 | 150 | - | - | 150 | 150 | - |
| | | B4 | - | - | - | - | - | - | - | - | - | - |
| | | B5 | - | - | - | - | - | - | - | - | - | - |
| | | B6 | - | - | - | - | - | - | - | - | - | - |
| | | B7 | - | - | - | - | - | - | - | - | - | - |
| | | B8 | - | - | - | - | - | - | - | - | - | - |
| | Component (B') [mg/L] | B'1 | - | - | - | - | - | - | - | - | - | 150 |
| | Component (C) [mg/L] | C1 | - | - | - | - | - | - | - | - | - | - |
| | | C2 | - | - | - | - | - | - | - | - | - | - |
| | Component (E) | E1 | Proper Amount | Proper Amount | Proper Amount | - | Proper Amount | Proper Amount | Proper Amount | - | Proper Amount | Proper Amount |
| | | E2 | - | - | - | Proper Amount | - | - | - | Proper Amount | - | - |
| | pH | | 3.5 | 3.5 | 0.4 | 5.0 | 3.5 | 6.3 | 3.5 | 6.0 | 3.5 | 3.5 |
| Ozone Concentration [% by Volume] | | | 0.01 | 0.01 | 0.01 | 0.01 | - | 0.01 | 0.01 | 0.01 | 0.01 | 0.01 |
| Sterilizing Power Evaluation | Glass | | 1.8 | 0.8 | 3.3 | 0.4 | 0.2 | 0.6 | 0.4 | 0.6 | 1.8 | 1.7 |
| | SUS304 | | 1.9 | 1.5 | 4.0 | 0.9 | 0.7 | 0.6 | 0.9 | 0.6 | 1.8 | 1.8 |
| | Silicon Rubber | | 1.5 | 0.6 | 3.2 | 0.6 | 0.5 | 0.3 | 0.6 | 0.3 | 1.5 | 1.2 |
| Surface Deterioration Evaluation | Glass | | 4 | 4 | 4 | 4 | 4 | 4 | 4 | 4 | 4 | 4 |
| | SUS304 | | 4 | 4 | 1 | 4 | 4 | 4 | 4 | 4 | 4 | 4 |
| | Silicon Rubber | | 4 | 4 | 2 | 4 | 4 | 4 | 4 | 4 | 4 | 4 |
| Comprehensive Evaluation | | | X | X | X | X | X | X | X | X | X | X |

As shown in Table 2, in Examples 13 to 32 which used the sterilization auxiliary including the Component (A) and the Component (B), a high sterilizing effect was obtained even with a treatment for a short period of time with a small amount of ozone, for all of the test pieces of SUS304, silicon rubber, surface polished glass. In addition, in Examples 28 to 30 which used the sterilization auxiliary which included the Component (C) in addition to the Component (A) and the Component (B), a higher sterilizing effect was obtained due to the ozone-containing air bubbles being miniaturized.
Furthermore, in Examples 13 to 32, a large surface deterioration was not observed on any of the solid surfaces, therefore surface deterioration was also determined as a pass.

In addition, as shown in Table 3, in Comparative Example 12 which used the sterilization auxiliary which did not include the Component (B), in Comparative Example 13 which used the sterilization auxiliary which did not include the Component (A), and in Comparative Examples 15 which used the sterilization auxiliary which included the Component (A) and the Component (B), however, had a pH of 5.0 or more, sterilizing power was small for all of the test pieces.
Furthermore, in Comparative Example 14 which used the sterilization auxiliary which included the Component (A) and the Component (B), however, had a pH of less than 1.0, equal sterilizing power was exhibited compared to that of Example, however, surface deterioration was observed in SUS304 and silicon rubber.
In addition, in Comparative Example 16 which included the Component (A) and the Component (B), and in which the pH being was greater than or equal to 1.0 and less than 5.0, however, did not include ozone, and in Comparative Examples 17 to 19 using the sterilization auxiliary did not satisfy two of the three conditions of including the Component (A), including the Component (B) and the pH being greater than or equal to 1.0 and less than 5.0, sterilizing power became further smaller for all of the test pieces compared to those of Comparative Examples 12 to 15.
Furthermore, in Comparative Example 20 which included a metal salt of iron instead of the aluminum salts of the Component (A), the sterilizing power was smaller than that of examples and the effect of improving the sterilizing effect was not obtained.
In addition, In Comparative Example 21 which used L-ascorbic acid instead of the acid of the Component (B), the sterilizing power was smaller than that of examples and the effect of improving the sterilizing effect was not obtained.

### <Sterilization Test of Vegetables>

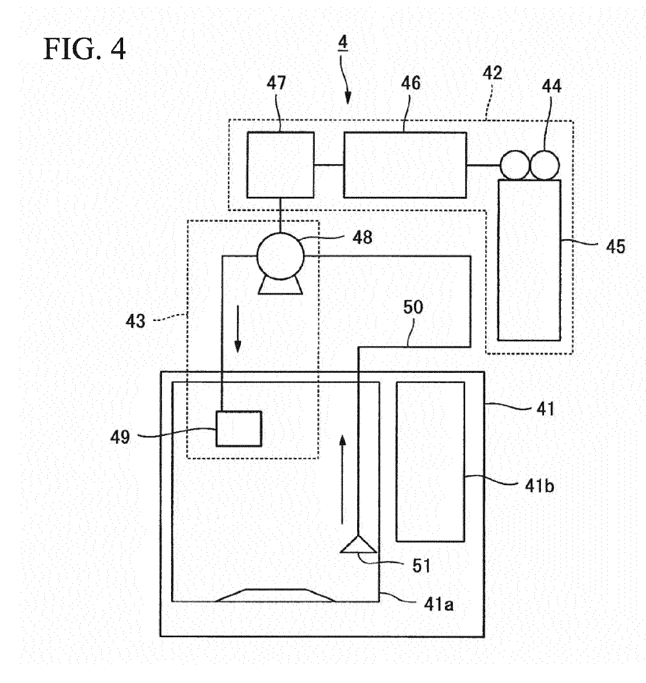

Sterilization washing of vegetables was carried out using the self-manufactured sterilization apparatus 4 exemplified in FIG 4.
As shown in FIG 4, the sterilization apparatus 4 is equipped with a two-tub washing machine 41 (manufactured by Mitsubishi Electric Corporation, CW-C30A1), ozone-containing gas supply means 42, and micro-air bubble generation means 43. The two-tub washing machine 41 has a washing tub 41a and a dewatering tub 41b. In the ozone-containing gas supply means 42, an air cylinder 45 in which a regulator 44 is installed, an ozone generator 46 (manufactured by Ebara Jitsugyo Co., Ltd., OZSD-3000A) and a mass flow controller 47 (manufactured by Kojima Instruments Inc., MODEL5100) are connected in this order. The micro-air bubble generation means 43 is equipped with a vortex pump 48 (manufactured by Nikuni Corporation, 20NED04) and a disperser 49 is connected at the distal end of the vortex pump. In the disperser 49, a cap is connected to both ends of a piece of cheese of 3/4 inches, and a hole of 3 mm is cut in the center. In addition, from the inside of the washing tub 41a of the two-tub washing machine 41 to the vortex pump 48, a piping 50 (made of vinyl chloride, Joint: SUS304) is disposed and at the end of the washing tub 41a side of the piping 50, a strainer 51 (a mesh made of Teflon (registered trademark) with a diameter of 1 mm) for preventing suction of vegetables is provided.
In the sterilization apparatus 4, ozone is generated in the ozone generator 46 using air sent from the air cylinder 45, and ozone-containing gas is supplied to the sterilization auxiliary supplied from the washing tub 41a to the vortex pump 48 through the piping 50 while the flow rate is adjusted by the mass flow controller 47. In the vortex pump 48, the sterilization auxiliary and the ozone-containing gas are gas-liquid mixed by the rotation of a impeller having a radial-shaped groove on the outer periphery (not shown), and the ozone-containing air bubbles are miniaturized using the disperser 49 and are supplied to the washing tub 41 a. The ozone-containing fine air bubbles with an average bubble diameter of approximately 50 µm may be generated by a shearing force between the vortex pump 48 and the disperser 49.

### [Examples 33 to 56]

Using 500 g of lettuce as the substance to be treated to be ozone sterilized, each operation of pre-washing, sterilization washing, rinsing, and dewatering was performed shown below with reference to general treatment of fresh food factories. Movement of the lettuce in each operation was carried out using a stainless steel colander sterilized with ethanol.

### (Pre-washing Step)

The lettuce was washed for 2 minutes using an electric bucket which stored 7 L of tap water (manufactured by Matsushita Electric Industrial Co., Ltd., N-Bk2).

### (Sterilization Step)

Ozone sterilization of the lettuce was performed for 10 minutes by collecting and stirring 40 L of the sterilization auxiliary in which each component was added to tap water in the composition shown in Table 4 in the washing tub 41a of the two-tub washing machine 41 in the sterilization apparatus 4 described above, and generating miniaturized ozone-containing bubbles. The pH adjustment of the sterilization auxiliary was carried out using a pH meter (manufactured by METTLER TOLEDO, Seven Easy). The ozone concentration in the ozone-containing gas supplied was monitored by an EG-600 manufactured by Ebara Jitsugyo Co., Ltd., and was adjusted to 0.5% by volume. The flow rate of the ozone-containing gas supplied from the ozone-containing gas supply means 42 was adjusted to 0.48 L/minute by a mass flow controller 47. In addition, the aeration conditions of the micro-air bubble generation means 43 were made to be 0.8 L/minute.

### (Rinsing Step)

The contents of the washing tub 41a were replaced with 40 L of tap water and the lettuce was rinsed while stirring for 5 minutes.

### (Dewatering Step)

The lettuce after the rinsing was transferred to the dewatering tub 41b and was dewatered for 1 minute.

### [Comparative Examples 22 to 31]

Ozone sterilization of the lettuce was performed in the same manner as that of Examples 33 to 56, except that the addition amount of each component of the sterilization auxiliary used and the pH were changed as shown in Table 5.

### [Evaluation Method of Sterilizing Power]

In the examination of the number of bacteria, 25 g of the lettuce before and after the ozone sterilization was added to 225 mL of buffer liquid containing peptone (a liquid in which 3.56 g of potassium dihydrogenphosphate, 18.2 g of disodium hydrogenphosphate dodecahydrate, 4.3 g of sodium chloride and 1.0 g of peptone were prepared in 1 L of purified water, and were neutralized to pH 7.0) and crushed by a stomacher equipped with a filter-attached stomacher bag and made to be a suspension. The above suspension was diluted in a stepwise manner and was poured into an SCD agar medium (Nissui Pharmaceutical Co., Ltd.). This was repeated three times per analyte, the number of colonies was counted after culturing for 24 hours at 36°C, and the number of viable bacteria was determined as the number of bacteria per 1 g of lettuce.
Evaluation of sterilizing power was performed using the multiple of the number of bacteria determined in each example (sterilization activity value) with respect to the number of bacteria when treated with 200 mg/L of sodium hypochlorite shown below (sterilization reference value). It should be noted that the above treatment is a standard sterilization method in the field of processing of cutting products of fresh vegetables. The lettuce was sterilized in the same manner as in Examples 33 to 56 except that instead of the auxiliary sterilization, the mixture was stirred for 5 minutes without ozone aeration using an aqueous solution of sodium hypochlorite with a concentration of 200 mg/L. The number of bacteria per 1 g of lettuce was measured in the same manner as the above measuring method for the lettuce after sterilization, and it was made to be a sterilization reference value.
In the present test, determination of a pass or a fail was made based on the value of sterilization activity value defined below. The number of bacteria is typically compared as a logarithmic axis, however, in the present test, the difference of the number of bacteria of the lettuce treated with 200 ppm of sodium hypochlorite with respect to the difference of the number of bacteria which is not converted to logarithm was calculated as a multiple and this value was made to be a sterilization activity value. If the sterilization activity value is "2.0", the difference of the number of bacteria with 200 ppm of sodium hypochlorite becomes "2.0" times and a decision to ensure equivalence to a treatment with 200 ppm of sodium hypochlorite becomes possible. In addition, in calculating the sterilization activity value of the present test, the number of bacteria in the treatment with 200 ppm of sodium hypochlorite may be either more or less than that of examples, and all were calculated to be positive value. In the present test, it was determined as pass if the sterilization activity value was less than "2.0" since such a result is practicable.

### [Evaluation Method of Appearance and Taste of Vegetables]

50 g of the lettuce after dewatering was spread out and placed on a white paper plate with a radius of 5 cm, and an appearance evaluation was performed visually. On the other hand, as an evaluation of taste, the lettuce after treatment was consumed and compared with untreated products. The evaluation criteria are shown below. As the following evaluation scores, points of 3 or more were determined as pass since such a result is practicable.

5 points: No changes were found in appearance and taste.
4 points: Slightly soft, but no change in taste.
3 points: Slightly soft and slight change in taste.
2 points: Slightly soft and considerable change in taste.
1 point: Quite soft and considerable change in taste.

For the sterilizing power evaluation of vegetables and the evaluation of appearance and taste, the results of Examples 33 to 56 are shown in Table 4, and the results of Comparative Examples 22 to 31 are shown in Table 5.

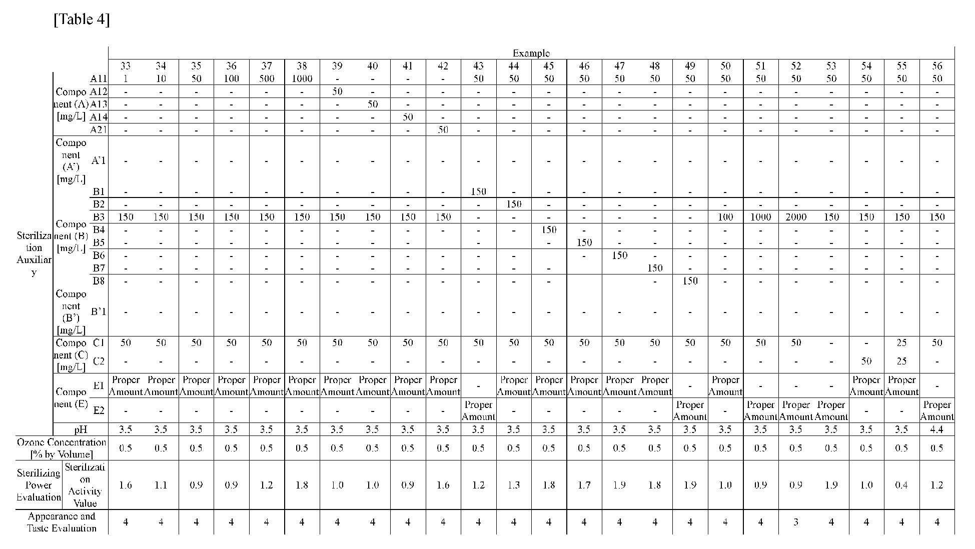

**[Table 5]**

| | | | | Comparative Example | | | | | | | | | |
|---|---|---|---|---|---|---|---|---|---|---|---|---|---|
| | | | | 22 | 23 | 24 | 25 | 26 | 27 | 28 | 29 | 30 | 31 |
| | Component (A) [mg/L] | | All | 50 | - | 50 | 50 | 50 | 50 | - | - | - | 50 |
| | | | A12 | - | - | - | - | - | - | - | - | - | - |
| | | | A13 | - | - | - | - | - | - | - | - | - | - |
| | | | A14 | - | - | - | - | - | - | - | - | - | - |
| | | | A21 | - | - | - | - | - | - | - | - | - | - |
| | Component (A') [mg/L] | | A'1 | - | - | - | - | - | - | - | - | 50 | - |
| | Component (B) [mg/L] | | B1 | - | - | - | - | - | - | - | - | - | - |
| | | | B2 | - | - | - | - | - | - | - | - | - | - |
| | | | B3 | - | 150 | 150 | 150 | 150 | - | - | 150 | 150 | - |
| | | | B4 | - | - | - | - | - | - | - | - | - | - |
| Sterilization Auxiliary | | | B5 | - | - | - | - | - | - | - | - | - | - |
| | | | B6 | - | - | - | - | - | - | - | - | - | - |
| | | | B7 | - | - | - | - | - | - | - | - | - | - |
| | | | B8 | - | - | - | - | - | - | - | - | - | - |
| | Component (B') [mg/L] | | B'1 | - | - | - | - | - | - | - | - | - | 150 |
| | Component (C) [mg/L] | | C1 | 50 | 50 | 50 | 50 | 50 | 50 | 50 | 50 | 50 | 50 |
| | | | C2 | - | - | - | - | - | - | - | - | - | - |
| | Component (E) | | E1 | Proper Amount | Proper Amount | Proper Amount | - | Proper Amount | - | Proper Amount | - | Proper Amount | Proper Amount |
| | | | E2 | - | - | - | Proper Amount | - | - | - | Proper Amount | - | - |
| | pH | | | 3.5 | 3.5 | 0.4 | 5.0 | 3.5 | 6.0 | 3.5 | 6.0 | 3.5 | 3.5 |
| Ozone Concentration [% by Volume] | | | | 0.5 | 0.5 | 0.5 | 0.5 | - | 0.5 | 0.5 | 0.5 | 0.5 | 0.5 |
| Sterilizing Power Evaluation | | Sterilization Activity Value | | 2.8 | 2.5 | 0.9 | 2.2 | 7.8 | 6.5 | 8.0 | 7.5 | 2.8 | 3.0 |
| Appearance and Taste Evaluation | | | | 4 | 4 | 2 | 4 | 4 | 4 | 4 | 4 | 4 | 4 |

As shown in Table 4, in Examples 33 to 53 which used the sterilization auxiliary including the Component (A) and the Component (B), a high sterilizing effect was obtained even with a treatment for a short period of time with a small amount of ozone. Furthermore, in Examples 33 to 53, the result of the appearance and taste of the lettuce after ozone sterilization was 3 points or more, therefore, it the result was practicable. In addition, when Examples 35 and 43 to 49, which used the sterilization auxiliary including different types of the Component (B) in the same amount, were compared, in cases in which acetic acid, phosphoric acid and citric acid were used as the Component (B) (Examples 35, 43 and 44), a higher sterilizing effect was obtained, and in a case in which acetic acid was used as the Component (B), the highest sterilizing effect was obtained. In addition, in Example 55 which used the sterilization auxiliary in which Monocaprylin and triacetin were combined as the Component (C), a particularly high sterilizing effect was obtained.

On the other hand, as shown in Table 5, in Comparative Example 22 which used the sterilization auxiliary which did not include the Component (B), in Comparative Example 23 which used the sterilization auxiliary which did not include the Component (A), and in Comparative Example 25 which used the sterilization auxiliary which included the Component (A) and the Component (B), however, had a pH of 5.0 or more, sterilizing power was smaller compared to those of the examples.
Furthermore, in Comparative Examples 24 which used the sterilization auxiliary which included the Component (A) and the Component (B), however, had a pH of less than 1.0, sterilizing power was exhibited, however, deterioration was observed in appearance and taste.
In addition, in Comparative Examples 26 in which the Component (A) and the Component (B) were included, and the pH was greater than or equal to 1.0 and less than 5.0, however, ozone was not included, the sterilizing power was extremely small.
Furthermore, in Comparative Examples 27 to 29 which used the sterilization auxiliary in which two of the three conditions of including the Component (A), including the Component (B) and the pH being greater than or equal to 1.0 and less than 5.0 were not satisfied, the sterilizing power became extremely small.
Furthermore, in Comparative Examples 30 which used ferrous sulfate instead of the aluminum salts of the Component (A), the sterilizing power was smaller than that of examples and the effect of improving the sterilizing effect was not obtained.
In addition, in Comparative Example 31 which used L-ascorbic acid instead of the acid of the Component (B), the sterilizing power was smaller than that of examples and the effect of improving the sterilizing effect was not obtained.

### Industrial Applicability

According to the ozone sterilization method using the sterilization auxiliary of the present invention, costs may be reduced since the use of additional facilities is not required, and the load on the sterilization apparatus may also be reduced since a higher sterilizing effect may be obtained with less ozone. Therefore, the sterilization auxiliary and the ozone sterilization method of the present invention may be suitably used in the sterilization of fresh food and medical equipment, or the stationary sterilization cleaning of an industrial plant line, or the like.

### Reference Signs List

1 sterilization apparatus 11 water tank 12 ozone-containing gas supply means 13 aeration means 14 supply pipe 15 air diffusion unit 16 ozone-containing air bubble 17 stirring means 18 substance to be treated

## Claims

1. A sterilization auxiliary for ozone sterilization which is an aqueous solution comprising:
a Component (A) of an aluminum compound which produces aluminum ions in an aqueous solution, and
a Component (B) of one or more of acids selected from phosphoric acid, acetic acid, citric acid, malic acid, succinic acid, gluconic acid, lactic acid, and L-tartaric acid,
wherein the pH of the aqueous solution is greater than or equal to 1.0 and less than 5.0.

2. The sterilization auxiliary for ozone sterilization according to Claim 1,
wherein the Component (B) is one or more acids selected from the group consisting of phosphoric acid, citric acid and acetic acid.

3. The sterilization auxiliary for ozone sterilization according to Claim 1 or 2,
wherein the Component (A) is one or more aluminum compounds selected from the group consisting of aluminum potassium sulfate (AlK(SO₄)₂·12H₂O), burnt potassium alum (AlK(SO₄)₂), aluminum ammonium sulfate (AlNH₄(SO₄)₂·2H₂O) and burnt ammonium alum (AlNH₄(SO₄)₂).

4. The sterilization auxiliary for ozone sterilization according to any one of Claims 1 to 3, further comprising:
a Component (C) of a glycerin fatty acid ester in which a fatty acid having 1 to 10 carbon atoms and glycerin are ester-bonded.

5. An ozone sterilization method for ozone-treating a substance to be treated using the sterilization auxiliary for ozone sterilization according to any one of Claims 1 to 4.
